# EUROPÄISCHE PATENTSCHRIFT

(11) **EP 0 885 394 B1**
(45) Veröffentlichungstag und Bekanntmachung des Hinweises auf die Patenterteilung: **16.08.2000**
(21) Anmeldenummer: 97904446.8
(22) Anmeldetag: 01.03.1997
(51) Int. Cl.: G01N 33/86, C12Q 1/56

(54) **VERFAHREN ZUR EIGNUNGSPRÜFUNG VON FAKTOR VIII-HALTIGEN PROTEINFRAKTIONEN**
PROCESS FOR TESTING SUITABILITY OF PROTEIN FRACTIONS CONTAINING FACTOR VIII
PROCEDE POUR L'ESSAI DE QUALIFICATION DE FRACTIONS PROTEIQUES CONTENANT LE FACTEUR VIII

(30) Priorität: 08.03.1996 DE 19609050; 10.05.1996 DE 19618851
(43) Veröffentlichungstag der Anmeldung: 23.12.1998
(73) Patentinhaber: Octapharma AG, 8853 Lachen (CH)
(72) Erfinder: BUCHACHER, Andrea, A-1180 Wien (AT); STADLER, Monika, A-2435 Wienerherberg (AT); JOSIC, Djuro, A-1020 Wien (AT); SCHWINN, Horst, D-55129 Mainz (DE)
(74) Vertreter: Werner, Hans-Karsten, Dr.Dipl.-Chem.
(86) Internationale Anmeldenummer: EP9700703
(87) Internationale Veröffentlichungsnummer: WO9733178

(56) Entgegenhaltungen:
- EP-A- 0 202 853
- EP-A- 0 359 201
- EP-A- 0 412 466
- WO-A-96/02572
- DE-C- 4 337 573

## Beschreibung

Gegenstand der vorliegenden Erfindung ist ein Verfahren zur Eignungsprüfung von Faktor VIII-haltigen Proteinfraktionen, deren Weiterverarbeitungsverfahren einen Pasteurisierungsschritt beinhaltet sowie die Verwertung von als ungeeignet gefundenen Chargen.

Faktor VIII-Präparate werden überwiegend hergestellt aus Kryopräzipitat. Die Präparate der Anmelderin werden hergestellt durch eine Reinigung des wiederaufgelösten Kryopräzipitats mit Aluminiumhydroxid, einer Virusinaktivierung durch Lösungsmittel/Detergenzien und anschließende Anionenaustausch-Chromatographie. Aufgrund von verschärften Sicherheitsvorschriften für die Virusinaktivierung wurde zusätzlich ein Pasteurisierungsschritt durchgeführt, bei dem die Präparate nach der ersten Virusinaktivierung 10 Stunden bei 63°C ± 1°C in Gegenwart eines Stabilisators erhitzt werden.

Während die nur nach dem Lösungsmittel/Detergenzien-Verfahren virusinaktivierten Präparate seit Jahren problemlos appliziert worden sind, traten bei den Präparaten, die zusätzlich dem Pasteurisierungsschritt unterworfen worden waren, unerwartete und nicht vorhersehbare Inhibitorbildungen in mit Faktor VIII vorbehandelten Patienten auf. Diese Inhibitorbildungen traten nach ca. sechs Wochen oder später auf. Eingehende Untersuchungen führten zu dem Ergebnis, daß diese Inhibitorbildungen gehäuft aufgetreten waren bei Patienten, die mit einer bestimmten Charge bzw. mit Chargen bestimmter Herkunft des Kryopräzipitats behandelt worden sind.

Weitere intensive Untersuchungen haben zu dem Ergebnis geführt, daß nur diese Chargen geringe Mengen aber durchaus nachweisbare und identifizierbare Mengen von Faktor VIII-Bruchstücken aufwiesen, die im Bereich von 20 bis 50 kD liegen. Diese Bruchstücke waren in keiner anderen Charge und auch nicht in Produkten von Wettbewerbern zu finden.

Bei der Auffindung dieser Zusammenhänge stellte sich weiterhin heraus, daß das frühe Auftreten der Inhibitorbildung nach ca. sechs Wochen nur bei den Patienten beobachtet worden war, die ausschließlich mit den Präparaten aus bestimmten Chargen behandelt worden waren, während Patienten mit einem späteren Auftreten dieser Inhibitorbildung mit Präparationen verschiedener Chargen behandelt worden waren. Es konnte somit mit sehr großer Sicherheit die Schlußfolgerung gezogen werden, daß diese Verunreinigungen in den zum Einsatz gekommenen Faktor VIII-haltigen Proteinfraktionen, nämlich Kryopräzipitat einer bestimmten Herkunftsquelle, die Inhibitorbildungen hervorruft. Dabei wurde weiterhin festgestellt, daß diese Inhibitorbildung nicht aufgetreten ist bei den Patienten, die bereits seit Jahren mit Präparaten der gleichen Herkunft behandelt worden sind, bei denen jedoch der Pasteurisierungsschritt noch nicht durchgeführt wurde. Es ist somit jetzt davon auszugehen, daß diese Bruchstücke des Faktor VIII beim Pasteurisierungsschritt Veränderungen erleiden, die dann die Inhibitorbildung verursachen.

Die Erfindung hat sich somit zur Aufgabe gemacht, eine Eignungsprüfung von Faktor VIII-haltigen Proteinfraktionen zu entwickeln, deren Weiterverarbeitungsverfahren einen Pasteurisierungsschritt beinhaltet. Die Aufgabe wird überraschend einfach dadurch gelöst, daß sämtliches Ausgangsmaterial untersucht wird auf Bruchstücke im Bereich von 20 bis 50 kD. Liegen derartige Bruchstücke nicht vor, kann dieses Ausgangsmaterial zur Herstellung eines zweifach virusinaktivierten Produktes eingesetzt werden. Sind hingegen derartige Bruchstücke vorhanden, sind zusätzliche Maßnahmen notwendig, um dieses Ausgangsmaterial zu brauchbaren und nebenwirkungsfreien Präparationen zu verarbeiten.

Eine Möglichkeit zur Weiterverarbeitung besteht, beispielsweise in der Verwendung dieses Ausgangsmaterials für das Verfahren gemäß deutscher Patentanmeldung 196 09 050.4. Dabei erfolgt nach einem chromatographischen Schritt an einem Anionenaustauschermaterial eine Größenausschlußchromatographie (size exclusion chromatography) an hydrophilen Materialien. Gegenstand der Erfindung ist somit auch die Verwendung von Chargen, die für das übliche Verfahren als ungeeignet befunden wurden.

Das Verfahren zur Eignungsprüfung von Faktor VIII-haltigen Proteinfraktionen, deren Weiterverarbeitungsverfahren einen Pasteurisierungsschritt beinhaltet, kann beispielsweise erfolgen mittels Elektrophorese oder Gelpermeations-Chromatographie. Enthält dieses Material praktisch keine Bruchstücke im Bereich von 20 bis 50 kD, können weitere Untersuchungen entfallen. Enthält das Material hingegen Bruchstücke in diesem Bereich, kann zusätzlich untersucht werden, ob es sich um Bruchstücke von Faktor VIII handelt. Dies kann beispielsweise erfolgen mit Hilfe von geeigneten Antikörpern. Derartige Antikörper sind im Handel erhältlich, beispielsweise von der Firma Seralab unter der Bezeichnung F VIII-HC (heavy chain) vom Klone 530 p.

Im Rahmen der Untersuchungen der Anmelderin wurde festgestellt, daß diese Bruchstücke des Faktor VIII dann entstehen, wenn das Ausgangsmaterial für Faktor VIII mit Thrombin oder anderen Proteasen reagieren konnte. Anderen Proteasen, die derartige Bruchstücke erzeugen, sind beispielsweise aktivierter Faktor IX oder aktivierter Faktor X. Faktor VIII besitzt ein Molekulargewicht von ca. 300 kD. Die handelsüblichen und gut verträglichen Präparationen enthalten darüber hinaus andere Proteine wie den von Willebrand Faktor. Sie enthalten aber nur Spuren von Produkten im Bereich unter 100 kD. In den Ausgangsmaterialien und Endprodukten der Chargen, die zu den Inhibitorbildungen geführt haben, fanden sich hingegen derartige Bruchstücke des Faktor VIII im Bereich zwischen 20 und 50 kD und teilweise sogar noch darunter. Derartige Bruchstücke finden sich hingegen im vermehrten Maße in Ausgangsmaterialien, die bewußt Thrombin oder anderen Proteasen ausgesetzt worden sind. Die kritischen Bruchstücke, insbesondere des Faktor VIII finden sich somit in Faktor VIII-haltigen Proteinfraktionen, die bei der Herstellung und Lagerung des Kryopräzipitats unzulässigen Bedingungen ausgesetzt waren.

Erfindungsgemäß ist es somit jetzt möglich, alle Faktor VIII-haltigen Proteinfraktionen einer einfachen Eignungsprüfung zu unterwerfen. Enthält das Ausgangsmaterial keine oder nur geringe Mengen von Bruchstücken im Bereich von 20 bis 50 kD, kann das Material bedenkenlos zur Herstellung von Faktor VIII-Präparaten verwendet werden, die einem Pasteurisierungsschritt unterworfen werden sollen. Die bisher üblichen Methoden der Elektrophorese oder Gelpermeations-Chromatographie sind in der Lage, Mengen derartiger Bruchstücke nachzuweisen, die über 1 % bezogen auf den Gehalt von Faktor VIII liegen. Mittels geeigneter Antikörper ist es weiterhin möglich, Bruchstücke des Faktor VIII zu identifizieren, die in Mengen von etwa 0,1 % bezogen auf den Faktor VIII vorliegen. Die Chargen an Kryopräzipitat, die zu der oben genannten Inhibitorbildung geführt haben, enthielten deutlich höhere Mengen derartiger Bruchstücke. Die Anwesenheit derartiger Bruchstücke ist somit bereits durch Elektrophorese und Gelpermeations-Chromatographie nachweisbar.

Die Verwendung und Weiterverarbeitung von Material, welches die Bruchstücke gemäß deutscher Patentanmeldung 196 09 050.4 enthält, erfolgt beispielsweise durch das Verfahren zur Herstellung eines hochreinen, virusfreien Antihämophiliefaktors (AHF oder Faktor VIII) mittels eines mehrstufigen chromatographischen Verfahrens, wobei, ausgehend von vorgereinigten, AHF enthaltenden Fraktionen oder Plasma, in einer Stufe nach der Virusinaktivierung eine Reinigung an mindestens einem Anionenaustauschermaterial erfolgt, wobei nach einem chromatographischen Schritt an dem Anionenaustauschermaterial eine Größenausschlußchromatographie (size exclusion chromatography) an hydrophilen Materialien erfolgt.

Ausgehend von AHF enthaltenden Fraktionen, die angereichert sein können, beispielsweise ausgehend von Kryopräzipitat oder aber auch Plasma, wird eine Virusinaktivierung durchgeführt. Eine Reinigung an Ionenaustauschermaterialien ist ebenfalls vorgesehen. Es schließt sich daran dann ein weiterer chromatographischer Reinigungsschritt an, und zwar als Größenausschlußchromatographie (size exclusion chromatography) an hydrophilen Materialien. Dieser Schritt weist den Vorteil auf, daß die danach gewonnenen Fraktionen sich durch einen Antihämophiliefaktor (Faktor VIII) hoher spezifischer Aktivität auszeichnen. Überraschenderweise wird durch diesen Schritt inaktives Material, das möglicherweise durch Denaturierung des aktiven Faktors bei der vorangegangenen Aufreinigung nach dem Stand der Technik entsteht, entfernt, ohne daß eine Ausbeuteminderung eintritt.

Vorzugsweise wird die Größenausschlußchromatographie (size exclusion chromatography) an Superose·-6 Material der Firma Pharmacia und/oder sogenannte Tentakel-Materialien der Firma Merck Fractogel· BioSec durchgeführt. Das letztgenannte Material ist insbesondere für eine effiziente Trennung bei höheren Flußraten geeignet. Durch Verkürzung der Trennzeiten ist die Tendenz zur Denaturierung der empfindlichen Proteine verringert und darüber hinaus weist dieses Material eine hohe Trennkapazität auf. Es kann insbesondere vorteilhaft sein, die Verwendung der genannten Materialien zu kombinieren, etwa indem zunächst eine Trennung an Fractogel· BioSec Materialien durchgeführt wird, gefolgt von einer Reinigung der so erhältlichen Fraktion an Superose·-6. Dabei wird vorzugsweise eine Anordnung verwendet, bei der die Materialien in Säulen gefüllt sind und hintereinander geschaltet werden.

Das Verfahren weist darüber hinaus den Vorteil auf, daß bei der Reinigung in Gegenwart von Calciumchlorid eine Trennung von Faktor VIII und von Willebrand Faktor (vWF) erreicht werden kann.

Das Verfahren ist sowohl für den präparativen als auch den analytischen Bereich geeignet. Vorzugsweise wird das Material, mit dem die Größenausschlußchromatographie durchgeführt wird, regeneriert. Die Regeneration erfolgt vorzugsweise mit einer alkalischen Lösung.

Die Größenausschlußchromatographie wird vorzugsweise mit einem Laufmittel mit einer Osmolalität von 300 bis 1.000 mOsmol/l, insbesondere 400 bis 800 mOsmol/l durchgeführt. Die Osmolalität wird vorzugsweise durch physiologisch unbedenkliche Salze wie Natriumchlorid und physiologisch verträgliche Puffersysteme wie Citratpuffer oder Histidin Hydrochlorid eingestellt. Der pH-Wert des Lösungsmittelsystems zur Durchführung der Größenausschlußchromatographie beträgt pH 7 bis 8.

Das so erhaltene Material ist bereits für die Anwendung am Patienten geeignet und weist gegenüber herkömmlichen Materialien den Vorteil auf, daß das so erhaltene Material eine hohe spezifische Aktivität aufweist und daher nicht mit inaktiven Proteinfraktion belastet ist.

Der nach dem Verfahren erhältliche Faktor ist aufgrund seiner höheren, im Stand der Technik nicht zu findenden Aktivität neu.

Üblicherweise enthält handelsübliches Faktor VIII-Präparat 1.000 internationale Einheiten (IE) Faktor VIII bestimmt nach dem Einstufentest oder chromogen. Ca. 300 bis 600 internationale Einheiten von Willebrand Faktor (vWF) gemessen nach dem immunologischen Test mit Anti-vWF-Antikörpern (ELISA) sind in handelsüblichen Präparaten enthalten. Der Proteingehalt solcher Präparate beträgt 5 bis 25 mg Protein, wobei ca. 20 bis 70 % auf die aktive Komponente, die aus einem Komplex aus Faktor VIII/vWF besteht. Der restliche Proteingehalt von 30 bis 80 % entfällt, je nach Charge, auf nicht aktive Komponenten, die entfernt werden können.

Das analytische Verfahren erfolgt folgendermaßen:

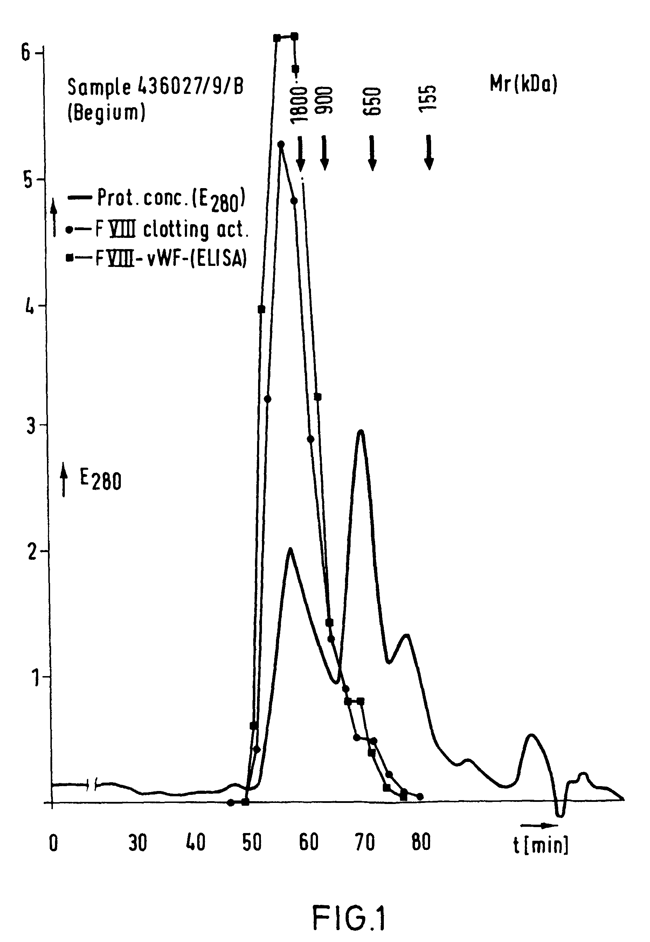

Die zu reinigende Probe wird in 4 ml bidestilliertem Wasser für Injektionen aufgelöst und enthält 250 IE Faktor VIII/ml. Als Trennsäule für die Größenausschlußchromatographie wird Superose 6, analytical grade, ausgewählt und in einer Säule mit 300 x 10 mm Innendurchmesser eingesetzt. Als mobile Phase wird 200 mM NaCl, gepuffert mit 50 mM Tris.HCl bei einem pH-Wert von 7,4 und einer Osmolalität von 450 bis 500 mOsmol/l verwendet. Die Säule wird zuvor mit 3 Säulenvolumina bidestilliertem Wasser gewaschen und gegebenenfalls regeneriert. Falls regeneriert wird, wird das Regenerationsmittel mit der korrespondierenden Säure oder Base neutralisiert und der gewünschte pH-Wert durch Waschen mit 3 Säulenvolumina 250 mM Tris.HCl bei pH 8,0 eingestellt. Danach wird die Säule mit 3 Säulenvolumina mobiler Phase äquilibriert. Die Flußrate der mobilen Phase beträgt ca. 0,5 ml/min. Die verwendete HPLC-Anlage besteht aus einer mikroprozessorgesteuerten Pumpe und einem UV-Spektralphotometer. Die Detektion gegebenenfalls durchfließender Komponenten erfolgt bei einer Wellenlänge von 280 nm. Die getrennten Fraktionen wurden anschließend mit einem Fraktionssammler gesammelt auf ihren Proteingehalt sowie auf Faktor VIII und vWF-Aktivität analysiert und danach mit SDS-Polyacrylamid Gelelektrophorese (SDS-PAGE) dargestellt. Die aufgegebene Probenmenge betrug 0,5 ml entsprechend einer Menge von 110 bis 150 internationalen Einheiten Faktor VIII. Die Trennung wurde bei Raumtemperatur bei einem Druck von 5 bis 15 bar durchgeführt. Die Wiedergewinnung des Faktors VIII belief sich auf 85 bis 95 %, diejenige des von Willebrand Faktors auf 70 bis 90 %. Das entsprechende Chromatogramm ist in Figur 1 gezeigt.

Die Trennung von Faktor VIII und von Willebrand Faktor in Anwesenheit von Calciumchlorid erfolgt folgendermaßen:

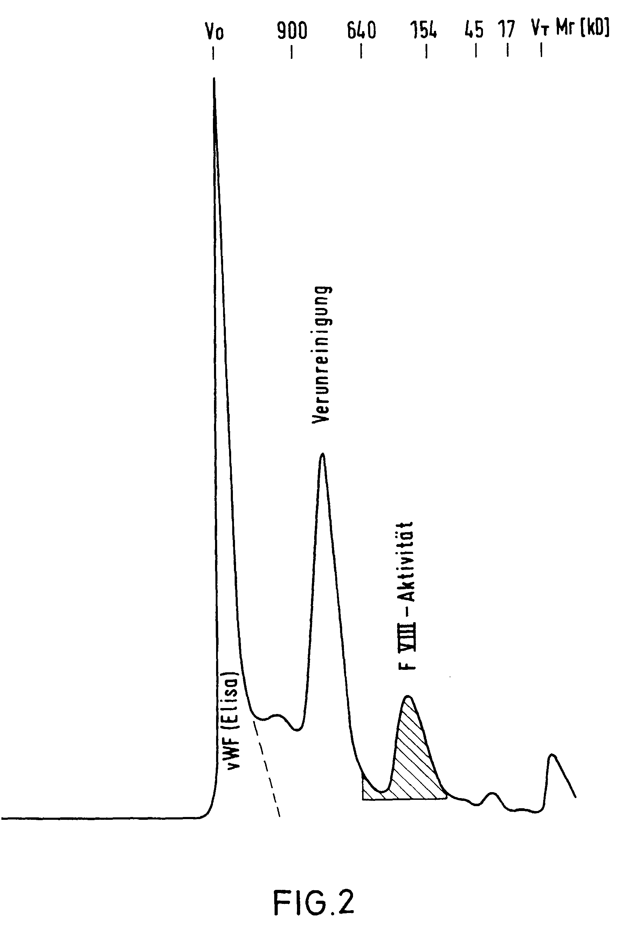

Der Komplex aus Faktor VIII/vWF zerfällt in Anwesenheit hoher Konzentrationen von Calciumchlorid. Zur Gewinnung von von Willebrand Faktor-freiem Faktor VIII kann der gereinigte Faktor VIII/vWF-Komplex in einer 250 mM Calciumchlorid Lösung dissoziiert werden. Die darauffolgende Trennung kann aufgrund der Molekülgröße durch Größenausschlußchromatographie erfolgen. Die Faktor VIII/vWF Komplex haltige Probe wird von nicht aktiven Proteinen gereinigt und gegen 250 mM Calciumchlorid-Lösung dialysiert. Die Konzentration betrug 250 IE Faktor VIII/ml und 150 IE vWF/ml. Die aufgegebene Proteinmenge betrug 500 µl. Es wurde eine mit Superose 6 (Analytical Grade) gefüllte, 300 x 10 mm Innendurchmesser aufweisende Säule verwendet. In diesem Falle diente als mobile Phase eine Lösung von 250 mM Calciumchlorid, 25 mM Tris.HCl bei einem pH-Wert von 7,4. Die Flußrate betrug 0,5 ml/min. Es wurde die gleiche obengenannte Anlage verwendet. Die Wiedergewinnung an Faktor VIII betrug 70 bis 80 % und an von Willebrand Faktor 85 bis 95 %. Das Chromatogramm ist in Figur 2 gezeigt.

Semipräparative und präparative Trennungen erfolgen folgendermaßen:

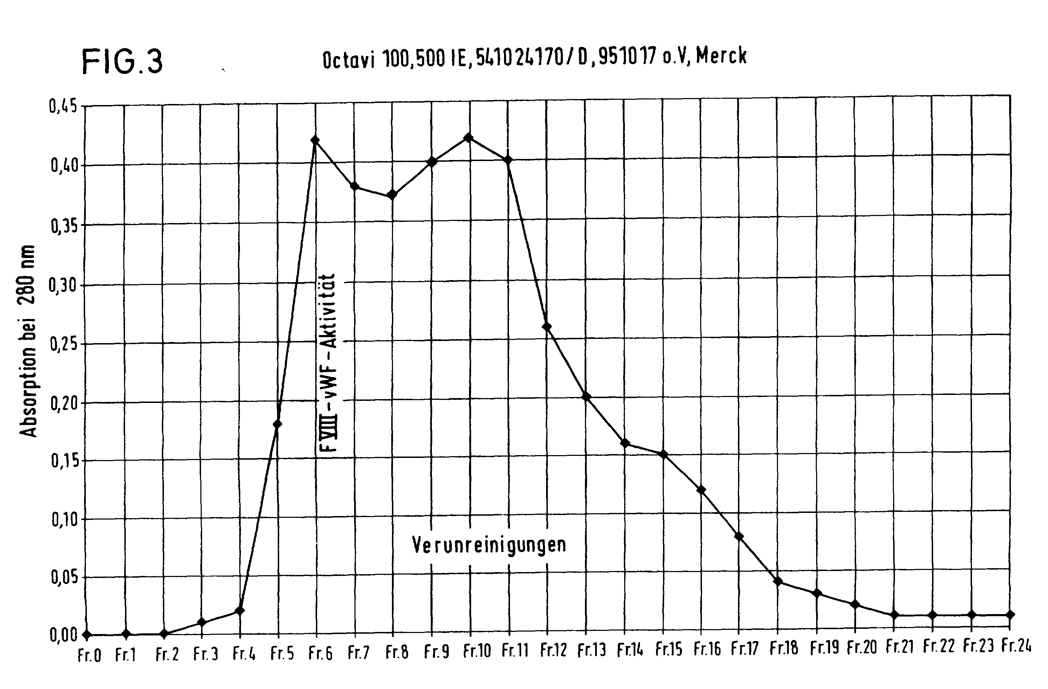

Die oben beschriebenen Trennungen können auch im semipräparativen bzw. präparativen Maßstab durchgeführt werden. Als Materialien kommen hier Fractogel BioSEC und Superose 6 (präparative Säule) in Frage. Die mit den Materialien erhaltenen Ergebnisse sind in Figuren 3 bis 5 gezeigt.

Die Figur 3 zeigt eine Größenausschlußchromatographie eines Faktors VIII/vWF-Komplexes im Gemisch mit aktiven Proteinen auf eine semipräparativen Säule mit Fractogel BioSEC (Tentakel)-Träger. Die Trennbedingungen sind wie folgt:
- Säulendimension:: 600 x 26 mm (Betthöhe: 570 mm)
- Mobile Phase:: 200 mM NaCl, 50 mM Tris.HCl, pH 7,4
- Osmolalität:: 450 bis 500 mOsmol/l
- Anlage:: Niederdruckpumpe (Pharmacia) und Photometer mit einer fixen Wellenlänge von 280 nm und einer präparativen Zelle. Die Fraktionen wurden gesammelt und ausgewertet wie in den Beispielen 1 und 2 beschrieben. Die Flußrate betrug 1,5 ml/min. beim einem Druck von weniger als 2 bar bei Raumtemperatur. Das Probenvolumen betrug 8 ml mit 2000 internationalen Einheiten Faktor VIII und 1280 internationalen Einheiten von Willebrand Faktor. Die Ausbeute betrug 90 bis 95 % Faktor VIII und 80 bis 90 % von Willebrand Faktor.

Größenausschlußchromatographie an Superose 6 (Prep-Grade)-Träger kann folgendermaßen durchgeführt werden:

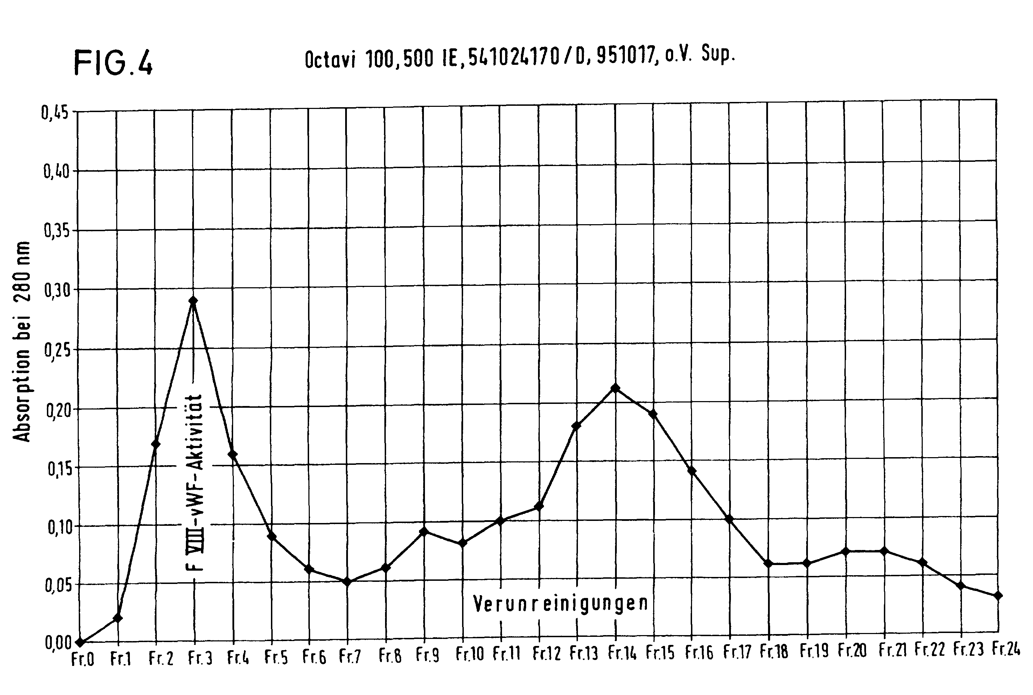

Eine Trennung wurde durchgeführt an einem Material Superose 6 (Prep Grade). Die Säulendimensionen betrugen 600 x 26 mm (Betthöhe 550 mm), die Flußrate betrug 1,5 ml/min., der Druck 2 bis 4 bar. Das Probenvolumen entsprach dem Obengenannten. Die Ausbeute betrug 80 bis 95 % Faktor VIII und 80 bis 95 % von Willebrand Faktor. Wie aus der Figur 4 ersichtlich, ist das Trennergebnis besser als dasjenige gemäß Figur 3, da die Verunreinigungen in Form von Fremdproteinen stärker abgesetzt sind.

Größenausschlußchromatographie eines Faktor VIII/vWF-Komplexes im Gemisch mit nicht aktiven Proteinen:

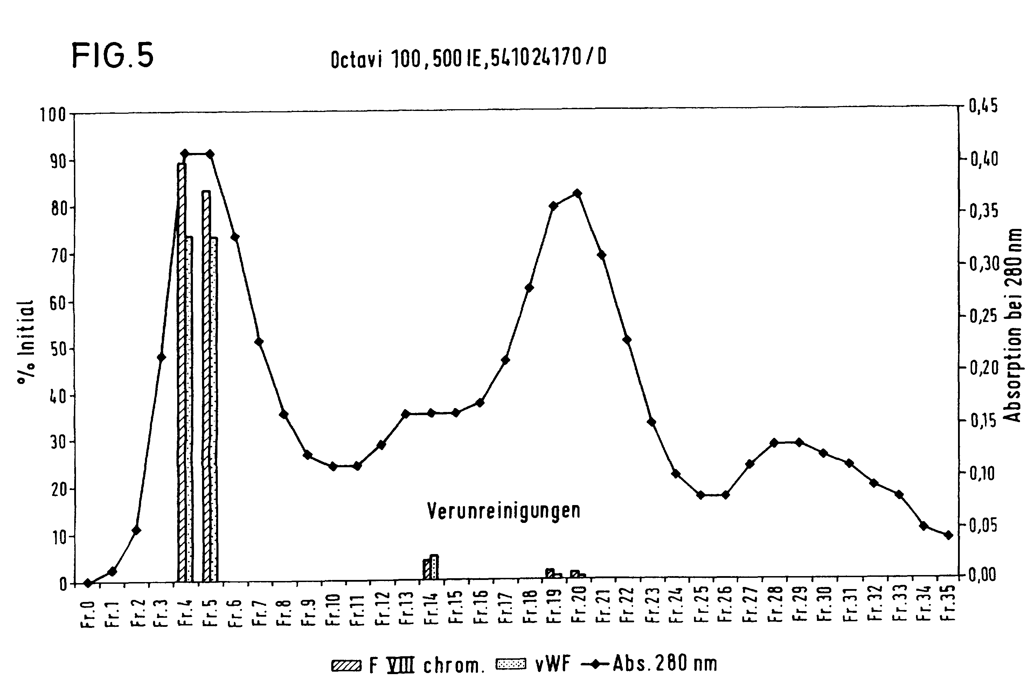

Die Größenausschlußchromatographie eines Faktors VIII/vWF-Komplexes im Gemisch mit nicht aktiven Proteinen auf einem Säulentandem bei dem je einer semipräparativen Fractogel BioSEC und einer Superose 6 (Prep-Grade)-Säule hintereinander geschaltet waren, wurde durchgeführt. Die Säulen entsprechen dem oben Beschriebenen. Die Flußrate betrug 1,5 ml/min bei einem Druck von weniger als 2 bar. Das Probenvolumen betrug 15 ml mit 4500 internationalen Einheiten Faktor VIII und 2600 internationalen Einheiten von Willebrand Faktor. Wie sich aus Figur 5 ergibt, ist die Trennung zwischen Faktor VIII/von Willebrand Komplex und Fremdproteinen sehr gut. Die Verwendung der Tandemsäule hat den Vorteil, daß Verstopfungen der Superose 6 Säule stark vermindert werden.

Semipräparative Abtrennung des Komplexes Faktor VIII/von Willebrand in Anwesenheit hoher Konzentration von Calciumionen erfolgt folgendermaßen:

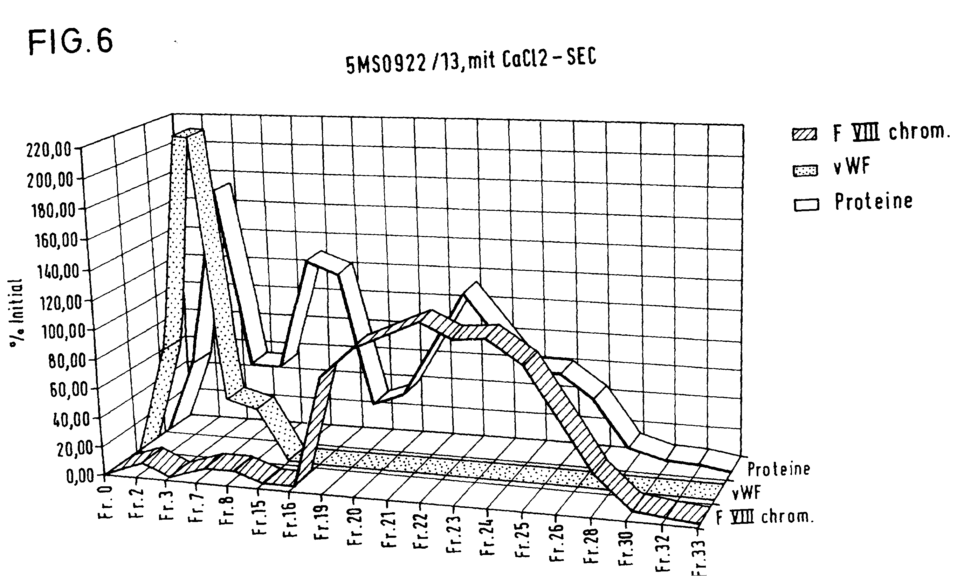

Es wird mit einem Säulentandem bestehend aus einer Fractogel BioSEC und einer Superose 6 (Prep-Grade)-Säule gearbeitet. Die semipräparative Trennung von Faktor VIII und von Willebrand Faktor erfolgt in Anwesenheit von 250 mM Calciumchlorid-Lösung. Es wurden 12 ml mit 3000 internationalen Einheiten Faktor VIII und 1800 internationalen Einheiten von Willebrand Faktor aufgetragen. Die mobile Phase war eine Lösung von 250 mM Calciumchlorid und 25 mM Tris.HCl bei pH 7,4. Die Wiedergewinnung von von Willebrand Faktor betrug 90 bis 95 % und von Faktor VIII 75 bis 90 %. Die Ergebnisse sind in Figur 6 dargestellt.

Eine Abtrennung von Detergenzien mit size exclusion chromatography bzw. Umpufferung erfolgt folgendermaßen:

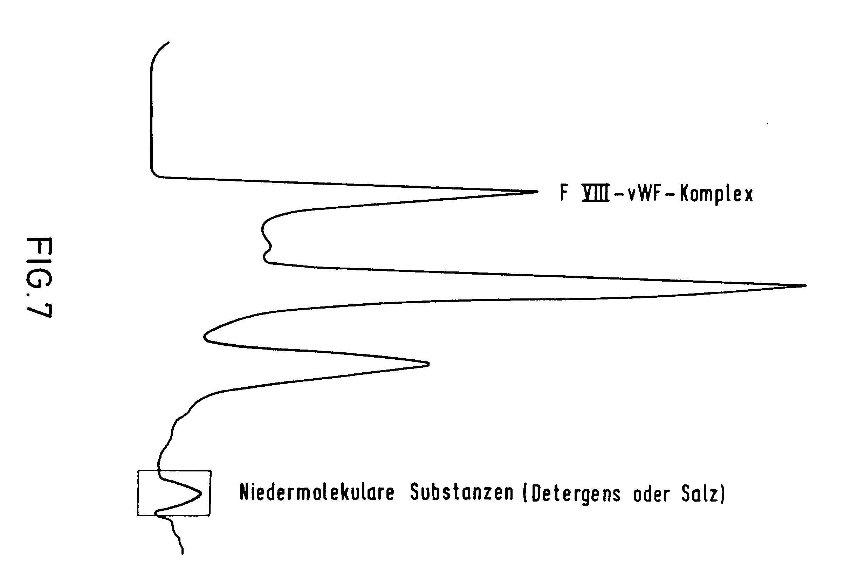

Die Figur 7 zeigt, daß Detergenzien bzw. Ionen durch die Größenausschlußchromatographie sehr vorteilhaft abgetrennt werden, so daß die nach der Größenausschlußchromatographie erhaltene Faktor VIII/von Willebrand-Komplex Fraktion nach Einstellung auf physiologische Bedingungen oder Auffangen und Gefriertrocknung direkt als Therapeutikum verwendet werden kann.

Die oben beschriebene Methode zur Reinigung von Faktor VIII und der Abtrennung von vWF kann auch für die weitere Reinigung von Faktor IX (FIX) benutzt werden. Bereits hoch vorgereinigtes FIX kann aufgetrennt werden in reineres FIX mit einer 3,6-fachen Steigerung der spezifischen Aktivität und hoher Ausbeute von mehr als 90 %. Das inaktive Material, abgetrennt vom FIX, enthält überwiegend ein Protein mit ungefähr 380 kDa. Das gereinigte FIX hat ungefähr 60 kDa und eine spezifische Aktivität von 340 IU/mg Protein. In einem Beispiel wird als Ausgangsmaterial 330 mg Protein verwendet (32.400 IU FIX). Erhalten wurden 29.500 IU (91 % der Theorie) von offensichtlich homogenem FIX. Der verwendete Puffer bestand aus 20 mM Na-Citrat (pH 7,4), 200 mM NaCl, 2 mM CaCl₂. Die Fließgeschwindigkeit betrug 5 cm/Stunde, der Druck 1-2 bar (0,1-0,2 MPa).

## Patentansprüche

1. Verfahren zur Eignungsprüfung von Faktor VIII-haltigen Proteinfraktionen, deren Weiterverarbeitungsverfahren einen Pasteurisierungsschritt beinhaltet, dadurch gekennzeichnet, daß das Ausgangsmaterial untersucht wird auf Bruchstücke im Bereich von 20 bis 50 kD.

2. Verfahren gemäß Anspruch 1, dadurch gekennzeichnet, daß untersucht wird auf Bruchstücke des Faktor VIII.

3. Verfahren gemäß Anspruch 2, dadurch gekennzeichnet, daß die Bruchstücke des Faktor VIII identifiziert werden mit Hilfe von geeigneten Antikörpern.

4. Verfahren gemäß einem der Ansprüche 1 bis 3, dadurch gekennzeichnet, daß die Untersuchung erfolgt mit Hilfe von Elektrophorese oder Gelpermeations-Chromatographie.

5. Verfahren gemäß einem der Ansprüche 1 bis 4, dadurch gekennzeichnet, daß Chargen, in denen die Bruchstücke aufgefunden wurden, verwendet werden zur Herstellung eines hochreinen, virusfreien Antihämophiliefaktors (AHF oder Faktor VIII) mittels eines mehrstufigen chromatographischen Verfahrens, wobei, ausgehend von vorgereinigten, AHF enthaltenden Fraktionen oder Plasma, in einer Stufe nach der Virusinaktivierung eine Reinigung an mindestens einem Anionenaustauschermaterial erfolgt, wobei nach einem chromatographischen Schritt an dem Anionenaustauschermaterial eine Größenausschlußchromatographie (size exclusion chromatography) an hydrophilen Materialien erfolgt.

## Claims

1. A method for the aptitude testing of protein fractions containing factor VIII the further processing of which comprises a pasteurizing step, characterized in that the starting material is examined for fragments within a range of from 20 to 50 kD.

2. The method according to claim 1, characterized in that the examination is directed to fragments of factor VIII.

3. The method according to claim 2, characterized in that said fragments of factor VIII are identified by means of appropriate antibodies.

4. The method according to any of claims 1 to 3, characterized in that the examination is performed by means of electrophoresis or gel permeation chromatography.

5. The method according to any of claims 1 to 4, characterized in that batches in which said fragments have been found are used for the preparation of a high purity virus-free antihemophilic factor (AHF or factor VIII) by means of a multi-step chromatographic method wherein, proceeding from prepurified, AHF-containing fractions or plasma, purification in one step on at least one anion exchanger material is performed following virus inactivation, wherein a chromatographic step performed on the anion-exchanger material is followed by size exclusion chromatography on hydrophilic materials.

## Revendications

1. Procédé pour l'essai de qualification de fractions protéiques contenant le facteur VIII, dont l'opération de post-transformation comporte une étape de pasteurisation, caractérisé en ce qu'on recherche, dans la matière de départ, les fractions comprises entre 20 et 50 kD.

2. Procédé selon la revendication 1, caractérisé en ce qu'on recherche des fractions du facteur VIII.

3. Procédé selon la revendication 2, caractérisé en ce que les fractions du facteur VIII sont identifiées à l'aide d'anticorps appropriés.

4. Procédé selon l'une des revendications 1 à 3, caractérisé en ce que la recherche s'effectue par électrophorèse ou par chromatographie par perméation de gel.

5. Procédé selon l'une des revendications 1 à 4, caractérisé en ce que l'on utilise des lots dans lesquels on a trouvé les fractions, pour préparer un facteur d'anti-hémophilie sans virus de haute pureté (AHF, ou facteur VIII) par une technique de chromatographie en plusieurs étapes, dans lequel, en partant de fractions ou de plasma prépurifiés et contenant de l'AHF, on procède dans une étape après l'inactivation du virus à une purification sur au moins un matériau échangeur d'anions, une chromatographie d'exclusion de taille étant réalisée sur des matériaux hydrophiles après une étape chromatographique sur le matériau échangeur d'anions.
